# EUROPÄISCHE PATENTSCHRIFT

(11) **EP 1 890 968 B1**
(45) Veröffentlichungstag und Bekanntmachung des Hinweises auf die Patenterteilung: **12.11.2008**
(21) Anmeldenummer: 06743172.6
(22) Anmeldetag: 13.06.2006
(51) Int. Cl.: C02F 1/04, C02F 1/20, C07D 251/60, C02F 101/38, C02F 101/16, C02F 103/36

(54) **VERFAHREN ZUR REINIGUNG VON ABWÄSSERN AUS MELAMINANLAGEN**
METHOD FOR THE PURIFICATION OF WASTEWATERS FROM MELAMINE SYSTEMS
PROCEDE D'EPURATION D'EAUX USEES PROVENANT D'INSTALLATIONS DE PRODUCTION DE MELAMINE

(30) Priorität: 15.06.2005 DE 102005028665
(43) Veröffentlichungstag der Anmeldung: 27.02.2008
(73) Patentinhaber: AMI Agrolinz Melamine International GmbH, 4021 Linz (AT)
(72) Erfinder: RUECH, Wolfgang, A-4753 Taiskirchen (AT); NEUMÜLLER, Christoph, A-4482 Ennsdorf (AT); WALLEK, Thomas, A-4400 Steyr (AT)
(74) Vertreter: Gross, Felix
(86) Internationale Anmeldenummer: PCT/EP2006/005847
(87) Internationale Veröffentlichungsnummer: WO 2006/133966

(56) Entgegenhaltungen:
- WO-A-01/46159
- WO-A-02/081379
- DE-A1- 1 930 844
- DE-A1- 10 229 103
- US-A- 4 013 757
- US-A- 4 663 387

## Beschreibung

Die Anmeldung betrifft ein Verfahren zur Reinigung von Abwässern aus Melaminanlagen gemäß Anspruch 1 sowie eine Vorrichtung gemäß Anspruch 21.

Die Herstellung von Melamin erfolgt nahezu ausschließlich aus Harnstoff nach folgender Reaktionsgleichung:

6 H₂N-CO-NH₂ → C₃N₃(NH₂)₃ + 6 NH₃ + 3 CO₂

Die Melaminherstellverfahren können in zwei Kategorien unterteilt werden. Es gibt nichtkatalytische Hochdruckverfahren, bei welchen das Melamin bei Drücken > 70 bar in flüssiger Phase hergestellt wird. Bei den katalytischen Niederdruckverfahren erfolgt die Melaminsynthese bei annäherndem Atmosphärendruck in der Gasphase.

Allen Melaminverfahren ist gemeinsam, dass das rohe Melamin aus der Synthesestufe anschließend gereinigt werden muss, da es Nebenprodukte enthält. Zum Abbau der Nebenprodukte wird das rohe Melamin meist in Gegenwart von Wasser aufgearbeitet, da die Nebenprodukte unter bestimmten Bedingungen in Wasser in Lösung gehen. Auf diese Weise fallen in verschiedenen Stufen der Melaminanlage mit stickstoffhaltigen Stoffen belastete Abwässer an. Bei diesen Abwasserinhaltsstoffen handelt es sich hauptsächlich um zyklische Stickstoffverbindungen in Form von Triazinen wie Melamin, Ureidomelamin, Cyanursäure oder Oxoaminotriazine (OAT) wie Ammelin oder Ammelid. Darüber hinaus können je nach Anlagenzustand variierende Mengen an azyklischen Stickstoffverbindungen wie beispielsweise Harnstoff oder auch Carbonate und Natrium- oder Ammoniumionen enthalten sein.

Die so belasteten Abwässer müssen zur Abtrennung ihrer Inhaltsstoffe behandelt werden, ehe sie ausgeschleust werden können.

Ein Beispiel für die Abwasserbehandlung eines Melaminverfahrens beschreibt die WO 01/46159 A2. Demnach wird die bei der Kristallisation des Melamins aus wässriger Lösung erhaltene triazin-belastete Mutterlauge angesäuert, wodurch die OAT auskristallisieren. Die so erhaltene OAT-Suspension wird einer Tangentialfiltration unterzogen, in welcher melaminreiches Permeat und als Retentat eine OAT-Suspension erhalten wird. Während das melaminreiche Permeat in die Anlage zurückgeführt wird, werden die OAT aus dem Retentat abgetrennt. Bei diesem Verfahren besteht der Nachteil, dass es aufwendig ist und darüber hinaus die ausgefällten OAT entsorgt werden müssen.

Eine weitere Möglichkeit der Abwasserbehandlung in Melaminanlagen besteht darin, die Abwässer in einer thermischen Abwasseraufarbeitungsanlage (TAA) zu behandeln, wo die triazinhältigen Abwasserinhaltsstoffe unter hohem Druck und hoher Temperatur in flüssiger Phase zu CO₂ und NH₃ hydrolisiert werden. Ein solches Verfahren ist beispielsweise in der IT 01282370 beschrieben. Dort werden die Kristallisationsmutterlaugen einer Melaminanlage in einem geschlossenen Gefäß unter dem Eigendruck des Systems auf 180 bis 250 °C erhitzt und für 20 bis 120 min belassen, wodurch die Inhaltsstoffe Melamin und OAT abgebaut werden.

Auf ähnliche Weise wird gemäß IT 0128369 triazinhältiges Melaminabwasser im geschlossenen Gefäß bei einer Temperatur von > 250°C behandelt. Das gebildete NH₃ und CO₂ wird anschließend abgestrippt und die erhaltene reine Flüssigkeit in die Anlage rückgeführt oder ausgeschleust.

Gemäß DE 102 29 103 A1 wird das triazinhältige Abwasser einer Melaminanlage in einer mäanderförmigen Strömung durch eine beheizbare Vorrichtung geleitet. Bei Temperaturen > 190 °C und dem Systemgleichgewichtsdruck, der bei etwa 30 bis 60 bar liegt, werden die Abwasserinhaltsstoffe in NH₃ und CO₂ zersetzt. Die Reaktion findet in der flüssigen Phase statt, wobei geringe Verdampfungsverluste nicht zu verhindern sind.

Den genannten Verfahren ist gemeinsam, dass sie einstufige Verfahren sind, deren Hydrolysevorrichtungen hinsichtlich Druck, Temperatur und Verweilzeit auf eine bestimmte, konstante Abwasserqualität und -menge ausgelegt sind. Im Anlagennormalbetrieb werden damit die gewünschten Abbaugrade der Inhaltsstoffe erreicht. Sobald jedoch Betriebszustände auftreten, bei welchen das Abwasser hinsichtlich Konzentration und Art der Inhaltsstoffe variiert, können die geforderten Abbaugrade nicht mehr gewährleistet werden.

Die Aufgabe der Erfindung bestand darin, ein Verfahren zur Reinigung von Abwässern einer Melaminanlage zu finden, welches die angeführten Nachteile nicht aufweist.

Gegenstand der vorliegenden Erfindung ist ein Verfahren zur Reinigung von Abwässern einer Melaminanlage, das dadurch gekennzeichnet ist, dass
- triazinhältiges Abwasser einer thermischen Vorbehandlungsstufe unterzogen wird, wobei eine Gasphase und eine Flüssigphase gebildet werden, worauf
- aus der Gasphase der thermischen Vorbehandlungsstufe die Brüden kondensiert werden, und
- die Flüssigphase der thermischen Vorbehandlungsstufe einer thermischen Hydrolysestufe unterzogen wird und aus der erhaltenen H₂O-, CO₂- und NH₃-hältigen Flüssigphase NH₃ abgetrennt wird.

Der Vorteil des erfindungsgemäßen zweistufigen Abwasserreinigungsverfahrens liegt darin, dass durch die thermische Vorbehandlungsstufe variierende Anlagenzustände, bei denen unterschiedliche Abwasserqualitäten auftreten, ausgeglichen werden können. In der thermischen Vorbehandlungsstufe werden bestimmte Abwasserinhaltsstoffe wie etwa Harnstoff bereits weitgehend zu NH₃ und CO₂ abgebaut. Solche harnstoffbelasteten Abwässer treten etwa bei Anfahr- oder auch Abfahrzuständen der Melaminanlage auf. Werden solche Abwässer ohne Vorbehandlungsstufe direkt in eine thermische Abwasserhydrolyse geführt, so muss die Betriebsweise der gesamten Anlage exakt auf die geänderten Bedingungen hin eingestellt werden um den gewünschten Abbaugrad der Abwasserinhaltsstoffe gewährleisten zu können. Dies erfordert einen hohen Regelungs- und Überwachungsaufwand in allen vom Normalbetrieb abweichenden Situationen.

Durch die erfindungsgemäß der Abwasserhydrolyse vorgeschaltete thermische Vorbehandlungsstufe ist es überraschenderweise möglich, variierende Abwasserqualitäten so weit auszugleichen, dass das Abwasser im Zulauf zur thermischen Hydrolyse nahezu unabhängig vom Anlagenzustand immer eine weitgehend gleiche Qualität aufweist. Dies erlaubt eine konstante und sichere Betriebsweise der Melamin- und der Abwasseranlage.

Ein weiterer Vorteil des erfindungsgemäßen Verfahrens darin, dass in der thermischen Vorbehandlungsstufe die zyklischen Stickstoffverbindungen bereits zu einem Teil abgebaut werden, so dass in der nachfolgenden Hydrolysestufe geringere Verweilzeiten für den Restabbau nötig sind.

So werden vorteilhafterweise in der thermischen Vorbehandlung mindestens 15 Gew-% des Abwassers in eine H₂O-, CO₂- und NH₃- hältige Gasphase überführt und separat aufgearbeitet, so dass die nachfolgende Hydrolysestufe auch hinsichtlich ihrer zu verarbeitenden hydraulischen Menge entlastet wird. Der restliche Teil des Abwassers wird als triazinhältige Flüssigphase erhalten.

Üblicherweise enthält das Abwasser einer Melaminanlage Triazine wie etwa die Oxoaminotriazine Ammelin und Ammelid, Melamin, Melam, Cyanmelamin, Ureidomelamin, Cyanursäure sowie Ammoniak, Kohlendioxid, Harnstoff und eventuell NaOH in unterschiedlichen Mengen.

Das Abwasser stammt zum Großteil aus der Melaminkristallisation und der Melaminfiltration der Melaminanlage und weist vorteilhafterweise die dort herrschenden pH-Werte und Temperaturen auf. Abwassertemperaturen von bis zu etwa 60 °C und pH Werte von etwa 12 sind dabei bevorzugt. Auch wird das Abwasser mit Vorteil vor seinem Eintritt in die thermische Vorbehandlungsstufe auf etwa 150 bis 250°C vorgewärmt.

Bevorzugt ist es, wenn das Abwasser aus einer Vakuumkristallisation und einer Melaminfiltration stammt. Besonders bevorzugt ist es, wenn das Abwasser aus einer einer Vakuumkristallisation nachgeschalteten Mutterlaugekristallisation stammt. In der Mutterlaugekristallisation wird aus der Mutterlauge der Melaminkristallisation das noch enthaltene Melamin weitgehend auskristallisiert. Erst die dort erhaltene nahezu melaminfreie Lösung gelangt in die Abwasseranlage, welche dadurch entlastet wird.

Die Temperaturen und Drücke in der thermischen Vorbehandlungsstufe können in weiten Bereichen variieren. Während die Temperatur über die Art und Menge des Wärmeträgers eingestellt wird, ist der Druck im Apparat von der Reaktionsgeschwindigkeit der ablaufenden Verdampfungs- und Abbaureaktionen sowie vom Dampfdruck der Reaktionsprodukte abhängig.

Bevorzugterweise beträgt die Temperatur in der thermischen Vorbehandlungsstufe 140 bis 250 °C, besonders bevorzugt 180 bis 220 °C.

Weiterhin bevorzugt beträgt der Druck in der thermischen Vorbehandlungsstufe 5 bis 50 bar, besonders bevorzugt 15 bis 30 bar.

Bei diesen Bedingungen erfolgt sowohl die Verdampfung des Abwassers als auch der Abbau einzelner Inhaltsstoffe so effizient, dass die Apparategröße in vernünftigen Grenzen gehalten werden kann. Über die Druck- und Temperatur-bedingungen kann auch die Wärmerückgewinnung aus den Brüden der thermischen Vorbehandlungsstufe beispielsweise in Form von Wasserdampf standortspezifisch optimiert werden.

Bevorzugterweise wird als Wärmeträger Dampf, beispielsweise Hochdruckdampf eingesetzt. Dampf steht in jeder Melaminanlage in ausreichender Menge zur Verfügung, wobei Dampf jeder beliebigen Druckstufe, auch etwa in Form von Mittel-oder Niederdruckdampf, zum Einsatz kommen kann.

Eine Hauptaufgabe der thermischen Vorbehandlungsstufe ist der Ausgleich unterschiedlicher Abwasserqualitäten durch den Abbau bestimmter Inhaltsstoffe, die insbesondere bei außergewöhnlichen Betriebszuständen der Melaminanlage auftreten. Diese Inhaltsstoffe sind oft azyklische Stickstoffverbindungen wie etwa Harnstoff, der verstärkt bei Anfahr- und Abfahroperationen auftritt. Es ist deshalb von Vorteil, wenn die triazinhältige Flüssigphase nach der thermischen Vorbehandlungsstufe nur mehr < 1 Gew-%, besonders bevorzugt 0,5 Gew-%, an azyklischen Stickstoffverbindungen aufweist. Auf diese Weise wird die nachfolgende thermische Hydrolysestufe soweit stofflich entlastet, dass sie praktisch immer untere konstanten Bedingungen betrieben werden kann.

Es ist von Vorteil, wenn die Verweilzeit des triazinhältigen Abwassers in der thermischen Vorbehandlungsstufe 0,5 bis 2 h, besonders bevorzugt 1 bis 1,5 beträgt.

Parallel zum Abbau von Abwasserinhaltsstoffen findet in der thermischen Vorbehandlungsstufe die Überführung eines Teils des Abwassers in die Gasphase statt. Dabei ist es umso vorteilhafter, je höher der in die Gasphase überführte Abwasseranteil ist. Ein hoher Gasphasenanteil bedeutet, dass große Wassermengen aus dem Abwasser verdampft werden und dass viele Nebenprodukte bereits in der Vorbehandlungsstufe abgebaut werden. Bevorzugt werden mindestens 50 Gew-% des Abwassers als H₂O-, CO₂- und NH₃-hältige Gasphase erhalten. Auf diese Weise können sowohl die hydraulische als auch die stoffliche Belastung für die nachfolgende thermische Hydrolysestufe bedeutend verringert werden.

Die in der thermischen Vorbehandlungsstufe entstehende Gasphase wird abgezogen und die Brüden werden kondensiert. Dabei erfolgt vorteilhafterweise eine Wärmerückgewinnung in Form von Wasserdampf. Je nachdem, welche Wasserdampfqualität am Standort verwertbar ist, kann beispielsweise Mitteldruckdampf oder Niederdruckdampf rückgewonnen gewonnen und in die Melaminanlage rückgeführt werden. Diese Wärmerückgewinnung verbessert die Gesamtdampfbilanz der Melaminanlage.

Die kondensierten Brüden der thermischen Vorbehandlungsstufe bestehen im Wesentlichen aus H₂O, CO₂ und NH₃. Sie können in die Harnstoff- oder in die Melaminanlage rückgeführt werden.

Bevorzugterweise werden aus den kondensierten Brüden CO₂ und NH₃ abgetrennt. Dies kann mittels bekannter Methoden, beispielsweise durch Strippen mit Dampf, erfolgen. Es wird reines Wasser erhalten, welches als Prozesskondensat in die Melaminanlage rückgeführt werden kann. Auf diese Weise kann zumindest ein Teil des benötigten Frischwassers eingespart werden.

Die in der thermischen Vorbehandlungsstufe erhaltene Flüssigphase enthält aufgrund der Abdampfung von Wasser und des Abbaus eines Teils der Inhaltsstoffe nahezu ausschließlich schwer abbaubare Verbindungen. Dies sind insbesondere die Triazine in Form der OATs Ammelin und Ammelin sowie Melamin.

Diese triazinhältige Flüssigphase wird einer thermischen Hydrolysestufe zugeführt, in welcher die Triazine im Wesentlichen in flüssiger Phase bei hohem Druck und hoher Temperatur zu H₂O, CO₂ und NH₃ abgebaut werden.

Bevorzugt findet die thermische Hydrolyse bei einer Temperatur von 200 bis 260 °C und einem Druck von 30 bis 100 bar statt. Üblicherweise ist die Temperatur der thermischen Hydrolysestufe höher als die Temperatur der thermischen Vorbehandlungsstufe, so dass der OAT-triazinhältigen Flüssigphase für die Hydrolyse Wärme zugeführt werden muss. Dies geschieht beispielsweise durch die Zufuhr von Dampf als Wärmeträger in die Hydrolysevorrichtung, wobei der Wärmeübergang üblicherweise in indirekter Form erfolgt.

Je höher die Temperatur in der thermischen Hydrolysestufe ist, desto rascher verläuft der Abbau der Abwasserinhaltsstoffe und desto höher ist der Druck, der nötig ist, um die Reaktionsmischung in flüssiger Phase zu halten.

Bevorzugt findet die thermische Hydrolyse in mindestens einem horizontalen Apparat statt. Eine vorteilhafte Variante einer thermischen Hydrolysestufe und der Hydrolysevorrichtung ist beispielsweise in der DE 102 29 103 A1 beschrieben. Es ist auch möglich für die thermische Hydrolyse, mehrere Apparate in Serie zu verwenden.

In der thermischen Hydrolysestufe wird eine H₂O-, CO₂- und NH₃-hältige Flüssigphase erhalten. Aus dieser Flüssigkeit wird vorteilhafterweise durch Strippen mit Dampf NH₃ abgetrennt, so dass am Sumpf des Strippers NH₃-freies H₂O abgezogen werden kann. Die NH₃-reichen Brüden werden in die Melamin,- oder in die Harnstoffanlage zurückgeführt.

Im speziellen Fall, dass der wässrige Aufarbeitungsteil der Melaminanlage in Gegenwart von NaOH erfolgt, wird die H₂O-reiche Phase Sumpfphase ausgeschleust; sie enthält unter anderem Na-Carbonat und kann deshalb nicht in den Melaminprozess rückgeführt werden.

Gegenstand der Erfindung ist auch eine Vorrichtung zur Durchführung des erfindungsgemäßen Verfahrens gemäß dem Anspruch 21.

Die Vorrichtung weist
- mindestens einen Apparat zur thermischen Vorbehandlung mit einem internen oder externen Wärmetauscher sowie einer Abscheidevorrichtung,
- mindestens einen Apparat zur Kondensation der Brüden aus der thermischen Vorbehandlungsstufe,
- mindestens einen Apparat zur thermischen Hydrolyse sowie
- mindestens einen Apparat zur Abtrennung von NH₃ aus der Flüssigphase der thermischen Hydrolysestufe
auf.

Mit dem vorliegenden Reinigungsverfahren kann das Abwasser jedes beliebigen Melaminverfahrens in einfacher und effizienter Weise gereinigt werden.

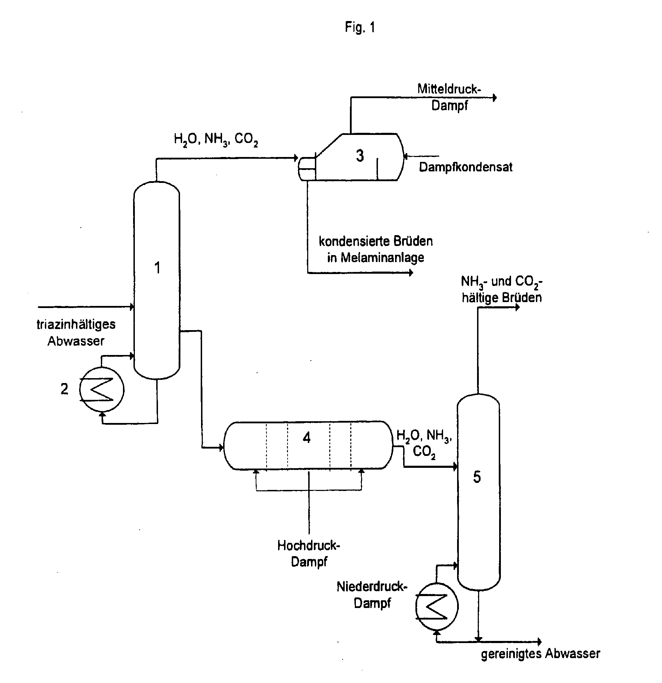

In Fig. 1 ist beispielhaft eine Ausführungsform des erfindungsgemäßen Verfahrens dargestellt.

Triazinhältiges Abwasser einer Melaminanlage wird einer thermischen Vorbehandlungsstufe 1 mit einem externen Wärmetauscher 2 zugeführt. Aus der thermischen Vorbehandlungsstufe 1 wird eine H₂O-, NH₃- und CO₂-hältige Gasphase abgezogen, welche im Dampferzeuger 3 mit Hilfe von Dampfkondensat kondensiert wird. Dabei wird Mitteldruckdampf gewonnen, der in die Melaminanlage rückgeführt wird.

Die Flüssigphase der thermischen Vorbehandlungsstufe 1 gelangt in die thermische Hydrolyse 4, wo in einem horizontalen Hydrolyseapparat mit Hilfe von Hochdruckdampf die Abwasserinhaltsstoffe abgebaut werden. Die H₂O-, CO₂- und NH₃-hältige Flüssigphase wird einem Abwasserstripper 5 zugeführt, wo durch Strippen mit Niederdruckdampf NH₃ und CO₂ ausgetrieben werden. Im Sumpf des Abwasserstrippers 5 wird gereinigtes Abwasser erhalten.

Im Folgenden wird die Erfindung anhand von Beispielen erläutert; die zugehörigen Messwerte sind in der Tabelle 1 dargestellt.

Im Vergleichsbeispiel wird Triazin und 4,0 Gew-% Harnstoff enthaltendes Abwasser aus einer Melaminanlage ohne thermische Vorbehandlungsstufe in eine thermische Hydrolysestufe, welche bei einer Temperatur von 240 °C und einem Druck von 45 bar betrieben wird, eingebracht. Das Reaktionsgemisch im Hydrolyseapparat enthält 4 Gew-% NH₃, 5,4 Gew.-% CO₂ sowie 90,6 Gew.-% H₂O. 20 Gew.-% des Reaktionsgemisches liegen gasförmig vor, wobei ein beträchtlicher Teil der Gasphase durch die Hydrolyse des Harnstoffes verursacht wird.

Um den Druck im Hydrolyseapparat konstant halten zu können, muss die Gasphase abgeführt werden. Das bedeutet, dass eine kontinuierliche Verdampfung stattfindet. Nachteilig dabei ist, dass durch die Bildung von Gasblasen das eigentliche Reaktionsvolumen für die Abbaureaktionen erheblich verringert und damit der Abbau der Abwasserinhaltsstoffe verschlechtert wird.

Alternativ zur Abführung der Gasphase kann zur Druckhaltung die Temperatur im Apparat erniedrigt werden, um den Gasphasenanteil geringer zu halten. Dadurch vermindern sich jedoch die Reaktionsgeschwindigkeiten, so dass lange Verweilzeiten nötig werden, um den gewünschten Abbaugrad der Triazine zu erreichen.

In Beispiel 1 wird dasselbe triazin- und harnstoffhältige Abwasser wie im Vergleichsbeispiel erfindungsgemäß zuerst einer thermischen Vorbehandlungsstufe und anschließend einer thermischen Hydrolysestufe unterzogen. Die thermische Vorbehandlung findet bei einer Temperatur von 200 °C, einem Druck von 22 bar für eine Verweilzeit von 1 h statt. Bei der thermischen Vorbehandlung werden 40 Gew-% der gesamten Abwassermenge verdampft. Der Großteil des im Abwasser enthaltenen Harnstoffes wird hier abgebaut und in Form von NH₃ und CO₂ entfernt. Die zurückbleibende flüssige Phase, welche eine hydraulische Menge von 60 Gew-% der gesamten Abwassermenge entspricht, enthält nur noch 2 Gew-% Harnstoff. Sie wird einer thermischen Hydrolysestufe bei denselben Temperatur- und Druckbedingungen wie im Vergleichsbeispiel unterzogen. Das Reaktionsgemisch im Hydrolyseapparat enthält 1,7 Gew-% NH₃, 1,1 Gew.-% CO₂ sowie 97,3 Gew.-% H₂O. Dies entspricht einem Gasphasenanteil von 2,3 Gew.-%. Das heißt, dass nur sehr geringe Verdampfung erfolgt und damit die Temperatur- und Druckbedingungen im Hydrolyseapparat konstant gehalten werden können. Dies führt zu den gewünschten hohen Abbaugeschwindigkeiten der Abwasserinhaltsstoffe.

### Vergleichsbeispiel: thermische Hydrolyse

| Reaktionsgemisch in thermischer | | |
|---|---|---|
| Hydrolysestufe | | |
| NH₃ | [Gew.%] | 4,0% |
| CO₂ | [Gew-%] | 5,4% |
| H₂O | [Gew.-% ] | 90,6% |
| | | |
| Temperatur | [°C] | 240 |
| Druck | [bar] | 45 |
| Gasphasenanteil | [Gew.-%] | 20% |

### Beispiel 1: thermische Vorbehandlung mit nachgeschalteter Hydrolyse

| Reaktionsgemisch in thermischer | | |
|---|---|---|
| Hydrolysestufe | | |
| NH₃ | [Gew.-%] | 1,7% |
| CO₂ | [Gew.- %] | 1,1% |
| Wasser | [Gew.-%] | 97,3% |
| | | |
| Temperatur | [°C] | 240 |
| Druck | [bar] | 45 |
| Gasphasenanteil | [Gew.-%] | 2,3% |

### Bezugszeichenliste

- 1: thermische Vorbehandlung
- 2: Wärmeaustauscher
- 3: Dampferzeuger
- 4: thermische Hydrolyse
- -5: Abwasserstripper

## Patentansprüche

1. Verfahren zur Reinigung von Abwässern einer Melaminanlage,
**dadurch gekennzeichnet, dass**
- triazinhältiges Abwasser einer thermischen Vorbehandlungsstufe unterzogen wird, wobei eine Gasphase und eine Flüssigphase gebildet werden, worauf
- aus der Gasphase der thermischen Vorbehandlungsstufe die Brüden kondensiert, und
- die Flüssigphase der thermischen Vorbehandlungsstufe einer thermischen Hydrolysestufe unterzogen wird und aus der erhaltenen H₂O-, CO₂- und NH₃-hältigen Flüssigphase NH₃ abgetrennt wird.

2. Verfahren nach Anspruch 1, **dadurch gekennzeichnet, dass** in der thermischen Vorbehandlungsstufe mindestens 15 Gew.-% des Abwassers als H₂O-, CO₂- und NH₃- hältige Gasphase und der restliche Teil des Abwassers als triazinhältige Flüssigphase erhalten werden.

3. Verfahren nach Anspruch 1 oder 2, **dadurch gekennzeichnet, dass** das triazinhältige Abwasser aus einer Vakuumkristallisation und einer Melaminfiltration stammt

4. Verfahren nach mindestens einem der vorhergehenden Ansprüche, **dadurch gekennzeichnet, dass** das triazinhältige Abwasser aus einer einer Vakuumkristallisation nachgeschalteten Mutterlaugekristallisation stammt.

5. Verfahren nach mindestens einem der vorher genannten Ansprüche, **dadurch gekennzeichnet, dass** die Temperatur in der thermischen Vorbehandlungsstufe 140 bis 250 °C beträgt.

6. Verfahren nach mindestens einem der vorher genannten Ansprüche, **dadurch gekennzeichnet, dass** die Temperatur in der thermischen Vorbehandlungsstufe 180 bis 220 °C beträgt.

7. Verfahren nach mindestens einem der vorher genannten Ansprüche, **dadurch gekennzeichnet, dass** der Druck in der thermischen Vorbehandlungsstufe 5 bis 50 bar beträgt.

8. Verfahren nach mindestens einem der vorher genannten Ansprüche, **dadurch gekennzeichnet, dass** der Druck in der thermischen Vorbehandlungsstufe 15 bis 30 bar beträgt.

9. Verfahren nach mindestens einem der vorher genannten Ansprüche, **dadurch gekennzeichnet, dass** der thermischen Vorbehandlungsstufe Dampf als Wärmeträger zugeführt wird.

10. Verfahren nach mindestens einem der vorher genannten Ansprüche, **dadurch gekennzeichnet, dass** die triazinhältige Flüssigphase aus der thermischen Vorbehandlungsstufe < 1 Gew.-% an azyklischen Stickstoffverbindungen aufweist.

11. Verfahren nach mindestens einem der vorher genannten Ansprüche, **dadurch gekennzeichnet, dass** die triazinhältige Flüssigphase aus der thermischen Vorbehandlungsstufe < 0,5 Gew.-% an azyklischen Stickstoffverbindungen aufweist.

12. Verfahren nach mindestens einem der vorher genannten Ansprüche, **dadurch gekennzeichnet, dass** die Verweilzeit des triazinhältigen Abwassers in der thermischen Vorbehandlungsstufe 0,5 bis 2 h beträgt.

13. Verfahren nach mindestens einem der vorher genannten Ansprüche, **dadurch gekennzeichnet, dass** die Verweilzeit des triazinhältigen Abwassers in der thermischen Vorbehandlungsstufe 1 bis 1,5 h beträgt.

14. Verfahren nach mindestens einem der vorher genannten Ansprüche, **dadurch gekennzeichnet, dass** in der thermischen Vorbehandlungsstufe mindestens 50 Gew.-% des Abwassers als H₂O-, CO₂- und NH₃-hältige Gasphase erhalten werden.

15. Verfahren nach mindestens einem der vorher genannten Ansprüche, **dadurch gekennzeichnet, dass** bei der Brüdenkondensation eine Wärmerückgewinnung in Form von Wasserdampf erfolgt und der Wasserdampf in die Melaminanlage rückgeführt wird.

16. Verfahren nach mindestens einem der vorher genannten Ansprüche, **dadurch gekennzeichnet, dass** aus den kondensierten Brüden der thermischen Vorbehandlungsstufe CO₂ und NH₃ durch Strippen mit Dampf abgetrennt werden und das erhaltene Wasser in die Melaminanlage rückgeführt wird.

17. Verfahren nach mindestens einem der vorher genannten Ansprüche, **dadurch gekennzeichnet, dass** die thermische Hydrolysestufe bei einer Temperatur von 200 bis 260 °C erfolgt.

18. Verfahren nach mindestens einem der vorher genannten Ansprüche, **dadurch gekennzeichnet, dass** die thermische Hydrolysestufe bei einem Druck von 30 bis 100 bar erfolgt.

19. Verfahren nach mindestens einem der vorher genannten Ansprüche, **dadurch gekennzeichnet, dass** die thermische Hydrolysestufe in mindestens einem horizontalen Apparat erfolgt.

20. Verfahren nach mindestens einem der vorher genannten Ansprüche, **dadurch gekennzeichnet, dass** aus der H₂O-, CO₂- und NH₃-hältigen Flüssigphase der thermischen Hydrolysestufe durch Strippen mit Dampf NH₃ abgetrennt und am Sumpf des Strippers NH₃-freies H₂O abgezogen wird.

21. Vorrichtung zur Durchführung des Verfahrens nach Anspruch 1,
**gekennzeichnet durch**
- mindestens einen Apparat zur thermischen Vorbehandlung aufweisend einen internen oder externen Wärmetauscher sowie eine Abscheidevorrichtung,
- mindestens einen Apparat zur Kondensation der Brüden aus der thermischen Vorbehandlungsstufe,
- mindestens einen Apparat zur thermischen Hydrolyse,
- mindestens einen Apparat zur Abtrennung von NH₃ aus der Flüssigphase der thermischen Hydrolysestufe.

## Claims

1. Process for cleaning wastewaters of a melamine plant,
**characterized in that**
- triazine-containing wastewater is subjected to a thermal pretreatment stage to form a gas phase and a liquid phase, then
- the vapours from the gas phase of the thermal pretreatment stage are condensed, and
- the liquid phase of the thermal pretreatment stage is subjected to a thermal hydrolysis stage and NH₃ is removed from the resulting H₂O-, CO₂- and NH₃-containing liquid phase.

2. Process according to Claim 1, **characterized in that** at least 15% by weight of the wastewater is obtained in the thermal pretreatment stage as H₂O-, CO₂- and NH₃-containing gas phase, and the remaining portion of the wastewater as triazine-containing liquid phase.

3. Process according to Claim 1 or 2, **characterized in that** the triazine-containing wastewater stems from a vacuum crystallization and a melamine filtration.

4. Process according to at least one of the preceding claims, **characterized in that** the triazine-containing wastewater stems from a mother liquor crystallization downstream of a vacuum crystallization.

5. Process according to at least one of the aforementioned claims, **characterized in that** the temperature in the thermal pretreatment stage is 140 to 250°C.

6. Process according to at least one of the aforementioned claims, **characterized in that** the temperature in the thermal pretreatment stage is 180 to 220°C.

7. Process according to at least one of the aforementioned claims, **characterized in that** the pressure in the thermal pretreatment stage is 5 to 50 bar.

8. Process according to at least one of the aforementioned claims, **characterized in that** the pressure in the thermal pretreatment stage is 15 to 30 bar.

9. Process according to at least one of the aforementioned claims, **characterized in that** steam is fed to the thermal pretreatment stage as a heat carrier.

10. Process according to at least one of the aforementioned claims, **characterized in that** the triazine-containing liquid phase from the thermal pretreatment stage has < 1% by weight of acyclic nitrogen compounds.

11. Process according to at least one of the aforementioned claims, **characterized in that** the triazine-containing liquid phase from the thermal pretreatment stage has < 0.5% by weight of acyclic nitrogen compounds.

12. Process according to at least one of the aforementioned claims, **characterized in that** the residence time of the triazine-containing wastewater in the thermal pretreatment stage is 0.5 to 2 h.

13. Process according to at least one of the aforementioned claims, **characterized in that** the residence time of the triazine-containing wastewater in the thermal pretreatment stage is 1 to 1.5 h.

14. Process according to at least one of the aforementioned claims, **characterized in that** at least 50% by weight of the wastewater are obtained in the thermal pretreatment stage as H₂O-, CO₂- and NH₃-containing gas phase.

15. Process according to at least one of the aforementioned claims, **characterized in that** heat is recovered in the vapour condensation in the form of steam and the steam is recycled into the melamine plant.

16. Process according to at least one of the aforementioned claims, **characterized in that** CO₂ and NH₃ are removed from the condensed vapours of the thermal pretreatment stage by stripping with steam and the resulting water is recycled into the melamine plant.

17. Process according to at least one of the aforementioned claims, **characterized in that** the thermal hydrolysis stage proceeds at a temperature of 200 to 260°C.

18. Process according to at least one of the aforementioned claims, **characterized in that** the thermal hydrolysis stage proceeds at a pressure of 30 to 100 bar.

19. Process according to at least one of the aforementioned claims, **characterized in that** a thermal hydrolysis stage proceeds in at least one horizontal apparatus.

20. Process according to at least one of the aforementioned claims, **characterized in that** NH₃ is removed by stripping with steam from the H₂O-, CO₂- and NH₃-containing liquid phase of the thermal hydrolysis stage and NH₃-free H₂O is drawn off at the bottom of the stripper.

21. Apparatus for performing the process according to Claim 1,
**characterized by**
- at least one apparatus for thermal pretreatment having an internal or external heat exchanger and a separation apparatus,
- at least one apparatus for condensation of the vapours from the thermal pretreatment stage,
- at least one apparatus for thermal hydrolysis,
- at least one apparatus for removing NH₃ from the liquid phase of the thermal hydrolysis stage.

## Revendications

1. Procédé pour la purification des eaux usées d'une installation de mélamine, **caractérisé en ce que**
- l'eau usée contenant de la triazine est soumise à une étape de prétraitement thermique, où sont formées une phase gazeuse et une phase liquide, puis
- on condense les vapeurs de la phase gazeuse de l'étape de prétraitement thermique, et
- on soumet la phase liquide de l'étape de prétraitement thermique à une étape d'hydrolyse thermique et on sépare du NH₃ de la phase liquide obtenue contenant H₂O, CO₂ et NH₃.

2. Procédé selon la revendication 1, **caractérisé en ce que** dans l'étape de prétraitement thermique, au moins 15% en poids de l'eau usée sont obtenus comme phase gazeuse contenant H₂O, CO₂ et NH₃ et la partie résiduelle de l'eau usée est obtenue comme phase liquide contenant de la triazine.

3. Procédé selon la revendication 1 ou 2, **caractérisé en ce que** l'eau usée contenant de la triazine provient d'une cristallisation sous vide et d'une filtration de mélamine.

4. Procédé selon au moins l'une quelconque des revendications susmentionnées, **caractérisé en ce que** l'eau usée contenant de la triazine provient d'une cristallisation de lessive-mère disposée en aval d'une cristallisation sous vide.

5. Procédé selon au moins l'une quelconque des revendications susmentionnées, **caractérisé en ce que** la température dans l'étape de prétraitement thermique est de 140 à 250°C.

6. Procédé selon au moins l'une quelconque des revendications susmentionnées, **caractérisé en ce que** la température dans l'étape de prétraitement thermique est de 180 à 220°C.

7. Procédé selon au moins l'une quelconque des revendications susmentionnées, **caractérisé en ce que** la pression dans l'étape de prétraitement thermique est de 5 à 50 bars.

8. Procédé selon au moins l'une quelconque des revendications susmentionnées, **caractérisé en ce que** la pression dans l'étape de prétraitement thermique est de 15 à 30 bars.

9. Procédé selon au moins l'une quelconque des revendications susmentionnées, **caractérisé en ce que** l'étape de prétraitement thermique est alimentée en vapeur comme support thermique.

10. Procédé selon au moins l'une quelconque des revendications susmentionnées, **caractérisé en ce que** la phase liquide contenant de la triazine provenant de l'étape de prétraitement thermique présente < 1% en poids de composés azotés acycliques.

11. Procédé selon au moins l'une quelconque des revendications susmentionnées, **caractérisé en ce que** la phase liquide contenant de la triazine provenant de l'étape de prétraitement thermique présente < 0,5% en poids de composés azotés acycliques.

12. Procédé selon au moins l'une quelconque des revendications susmentionnées, **caractérisé en ce que** le temps de séjour de l'eau usée contenant de la triazine dans l'étape de prétraitement thermique est de 0,5 à 2 h.

13. Procédé selon au moins l'une quelconque des revendications susmentionnées, **caractérisé en ce que** le temps de séjour de l'eau usée contenant de la triazine dans l'étape de prétraitement thermique est de 1 à 1,5 h.

14. Procédé selon au moins l'une quelconque des revendications susmentionnées, **caractérisé en ce qu'**on obtient, dans l'étape de prétraitement thermique, au moins 50% en poids de l'eau usée sous forme de phase gazeuse contenant H₂O, CO₂ et NH₃.

15. Procédé selon au moins l'une quelconque des revendications susmentionnées, **caractérisé en ce que** lors de la condensation des vapeurs, il se produit une récupération de chaleur sous forme de vapeur d'eau et la vapeur d'eau est recyclée dans l'installation de mélamine.

16. Procédé selon au moins l'une quelconque des revendications susmentionnées, **caractérisé en ce que** qu'on sépare des vapeurs condensées de l'étape de prétraitement thermique CO₂ et NH₃ par extraction à la vapeur et l'eau obtenue est recyclée dans l'installation de mélamine.

17. Procédé selon au moins l'une quelconque des revendications susmentionnées, **caractérisé en ce que** l'étape d'hydrolyse thermique est réalisée à une température de 200 à 260°C.

18. Procédé selon au moins l'une quelconque des revendications susmentionnées, **caractérisé en ce que** l'étape d'hydrolyse thermique est réalisée à une pression de 30 à 100 bars.

19. Procédé selon au moins l'une quelconque des revendications susmentionnées, **caractérisé en ce que** l'étape d'hydrolyse thermique est réalisée dans au moins un appareil horizontal.

20. Procédé selon au moins l'une quelconque des revendications susmentionnées, **caractérisé en ce qu'**on sépare de la phase liquide contenant H₂O, CO₂ et NH₃ de l'étape d'hydrolyse thermique, par extraction par la vapeur, NH₃ et qu'on soutire dans le fond de l'appareil d'extraction à la vapeur H₂O exempt de NH₃.

21. Dispositif pour la réalisation du procédé selon la revendication 1, **caractérisé par**
- au moins un appareil pour le prétraitement thermique présentant un échangeur de chaleur interne ou externe ainsi qu'un dispositif de séparation,
- au moins un appareil pour la condensation des vapeurs provenant de l'étape de prétraitement thermique,
- au moins un appareil pour l'hydrolyse thermique,
- au moins un appareil pour la séparation de NH₃ de la phase liquide de l'étape d'hydrolyse thermique.
